# EUROPEAN PATENT SPECIFICATION

(11) **EP 4 382 117 B1**
(45) Date of publication and mention of the grant of the patent: **02.12.2026**
(21) Application number: 22853181.0
(22) Date of filing: 05.08.2022
(51) Int. Cl.: A61K 38/20, A61K 39/395, A61K 45/00, A61P 35/00, A61P 43/00, C07K 16/18

(54) **COMBINATION TREATMENT FOR LIVER CANCER RESISTANT TO MONOTHERAPY WITH AN IMMUNE CHECKPOINT INHIBITOR OR A COMBINATION THERAPY WITH AN IMMUNE CHECKPOINT INHIBITOR AND IL-18**
KOMBINATIONSTHERAPIE FÜR LEBERKREBS, DER GEGEN EINE MONOTHERAPIE MIT EINEM IMMUNCHECKPOINT-INHIBITOR ODER EINE KOMBINATIONSTHERAPIE MIT EINEM IMMUNCHECKPOINT-INHIBITOR UND IL-18 RESISTENT IST
TRAITEMENT COMBINÉ POUR LE CANCER DU FOIE RÉSISTANT À LA MONOTHÉRAPIE PAR INHIBITEUR DE POINT DE CONTRÔLE IMMUNITAIRE OU À UN TRAITEMENT COMBINÉ PAR INHIBITEUR DE POINT DE CONTRÔLE IMMUNITAIRE ET IL-18

(30) Priority: 06.08.2021 JP 2021130294
(43) Date of publication of application: 12.06.2024
(73) Proprietor: NAGASAKI UNIVERSITY, Nagasaki 852-8521 (JP); Tokyo Metropolitan Hospital Organization, Shinjuku-ku Tokyo 163-8001 (JP)
(72) Inventor: TANAKA, Yoshimasa, Nagasaki-shi, Nagasaki 852-8521 (JP); KIMURA, Kiminori, Tokyo 113-8677 (JP)
(74) Representative: J A Kemp LLP
(86) International application number: PCT/JP2022/030084
(87) International publication number: WO 2023/013763

(56) References cited:
- WO-A1-2018/234862
- WO-A1-2020/264043
- US-A1- 2017 224 791
- US-A1- 2019 309 070
- Z. MA ET AL: "Augmentation of Immune Checkpoint Cancer Immunotherapy with IL18", CLINICAL CANCER RESEARCH, vol. 22, no. 12, 11 January 2016 (2016-01-11), US, pages 2969 - 2980, XP055392162, ISSN: 1078-0432, DOI: 10.1158/1078-0432.CCR-15-1655
- DONG WENJUAN, WU XIAOJIN, MA SHOUBAO, WANG YUFENG, NALIN ANSEL P., ZHU ZHENG, ZHANG JIANYING, BENSON DON M., HE KAI, CALIGIURI MIC: "The Mechanism of Anti–PD-L1 Antibody Efficacy against PD-L1–Negative Tumors Identifies NK Cells Expressing PD-L1 as a Cytolytic Effector", CANCER DISCOVERY, AMERICAN ASSOCIATION FOR CANCER RESEARCH, US, vol. 9, no. 10, 1 October 2019 (2019-10-01), US , pages 1422 - 1437, XP055958906, ISSN: 2159-8274, DOI: 10.1158/2159-8290.CD-18-1259
- SENJU HIROAKI, KUMAGAI ASUKA, NAKAMURA YOICHI, YAMAGUCHI HIROYUKI, NAKATOMI KATSUMI, FUKAMI SHOTA, SHIRAISHI KENGO, HARADA YUKA, N: "Effect of IL-18 on the Expansion and Phenotype of Human Natural Killer Cells: Application to Cancer Immunotherapy", INTERNATIONAL JOURNAL OF BIOLOGICAL SCIENCES, vol. 14, no. 3, 1 January 2018 (2018-01-01), pages 331 - 340, XP055820133, ISSN: 1449-2288, DOI: 10.7150/ijbs.22809
- RAHIMI KALATEH SHAH MOHAMMAD GHASEM, GHAHREMANLOO ATEFEH, SOLTANI ARASH, FATHI ESMAT, HASHEMY SEYED ISAAC: "Cytokines as potential combination agents with PD‐1/PD‐L1 blockade for cancer treatment", JOURNAL OF CELLULAR PHYSIOLOGY, WILEY SUBSCRIPTION SERVICES, INC., US, vol. 235, no. 7-8, 1 July 2020 (2020-07-01), US , pages 5449 - 5460, XP055930605, ISSN: 0021-9541, DOI: 10.1002/jcp.29491

## Description

### [Technical Field]

The present invention relates to therapeutic agents for cancer, containing T cell growth factor such as interleukin-2 (IL-2) and the like, interleukin-18 (IL-18), and immune checkpoint inhibitor in combination.

### [Background Art]

Non-drug therapies such as liver resection, liver transplantation, puncture local therapy, and Transcatheter Arterial Chemo-Embolization (TACE) are the main treatments for liver cancer. For progressive hepatocellular cancer for which these treatments are not indicated, drug therapy with vascular endothelial growth factor (VEGF) inhibitor sorafenib and lenvatinib is used. However, even with the latest drug, lenvatinib, the response rate is around 40%. Anti-PD-1 antibodies (nivolumab, pembrolizumab), which are immune checkpoint inhibitors, have recently been approved in the United States as second-line therapeutic drug for hepatocellular cancer in patients having history of sorafenib administration. However, pembrolizumab failed to meet the primary evaluation items of overall survival and progression-free survival in the phase III trial with placebo control. Combined use with VEGF inhibitor is also being studied, but the response rate for the combined use of pembrolizumab and lenvatinib is only around 60%, and it cannot be said to be sufficiently effective.

Inflammatory cytokines strongly activate T cells and natural killer (NK) cells, which are important for cancer immunity. Thus, various cytokine therapies have been investigated in expectation of antitumor effects. IL-18 markedly promotes the proliferation of NK cells, CD8-positive killer T cells (CTL), γδT cells, and the like. However, administration of IL-18 alone did not show sufficient effectiveness in Phase II trials for metastatic melanoma. The research group of the present inventors previously reported that the combined use of immune checkpoint inhibitor and IL-18 increases NK cells (helper NK cells) and CTLs, which are expected to have the ability to enhance acquired immunity, and the antitumor effect of immune checkpoint inhibitor is enhanced (Patent Literature 1, Non Patent Literature 1).

On the other hand, IL-2 is produced by activated T cells, NK cells, dendritic cells, and the like, and causes the proliferation and activation of cells such as CTL and NK cells that exhibit tumoricidal action. Thus, it is clinically used to treat metastatic renal cancer, malignant melanoma, and the like by single administration or in combination with NK cells activated by IL-2 (Non Patent Literature 2). The present inventors have found that when peripheral blood-derived NK cells are cultured in combination with IL-18 and IL-2, proliferation and activation of NK cells are markedly promoted (Non Patent Literature 3).

However, cytokine that exhibits an immunostimulatory action may also exhibit an action of promoting tumor growth. IL-2 induces the differentiation and activation of Th1, Th2, Th17, and regulatory T cells (Treg) from naive T cells, but Treg enhances suppression of CTL activity. Therefore, whether the effects in vitro can be reproduced by in vivo administration cannot be predicted at all. It has also been reported that IL-18 has dual properties: antitumor effect and tumor growth promoting effect (Non Patent Literature 4). It has also been reported that, in the cancer microenvironment, the expression of IL-18-binding protein (IL-18BP), which has extremely high affinity for IL-18, increased, and treatment with IL-18 or anti-PD-L1 antibody further increases expression of IL-18BP (Non Patent Literature 5). Therefore, it is unknown how effective the combined use of an immune checkpoint inhibitor and IL-18 is in actual human clinical practice.

Patent Literature 2 discloses methods of treating cancer in a subject including detecting an amount of PD-L1(+) natural killer cells in a biological sample from the subject and treating the subject with an anti cancer therapy.

Patent Literature 3 discloses IL-2 muteins that have an increased binding capacity for IL-2Rp receptor as compared to wild-type IL-2 for use in combination therapies with anti-PD-1 antibodies for the treatment of cancer.

Patent Literature 4 discloses compositions and methods relating to the treatment of a cancer via the induction of PD-L1 and use of anti-PD-L1 antibodies.

Patent Literature 5 discloses a cancer therapeutic agent that contains, as active ingredients, IL-18 and one or more antibodies selected from the group consisting of: an anti-PD-L1 antibody, an anti-PD-1 antibody, an anti-PD-L2 antibody, an anti-CTLA-4 antibody, an anti-CD25 antibody, an anti-CD33 antibody, and an anti-CD52 antibody.

Non Patent Literature 6 discloses that the mechanism of anti-PD-L1 antibody efficacy against PD-L1-negative tumors identifies NK cells expressing PD-L1 as a cytolytic effector.

Non Patent Literature 7 describes the effect of IL-18 on the expansion and phenotype of human natural killer cells and its application to cancer immunotherapy.

Non Patent Literature 8 discloses cytokines as potential combination agents with PD-1/PD-L1 blockade for cancer treatment.

### [Citation List]

### [Patent Literature]

[Patent Literature 1]
   JP-B- 6245622
[Patent Literature 2]
   WO 2020/264043
[Patent Literature 3]
   WO 2018/234862
[Patent Literature 4]
   US 2019/309070
[Patent Literature 5]
   US 2017/224791

### [Non Patent Literature]

[Non Patent Literature 1]
   Ma, Z. et al., Clin. Cancer Res., 22(12): 2969-2980 (2016)
[Non Patent Literature 2]
   Public Interest Incorporated Association, Japanese Society of Medical Oncology, "Cancer Immunotherapy Guideline" (KANEHARA & CO., LTD.), page 15, 20161220published
[Non Patent Literature 3]
   El-Darawish, Y. et al., J. Leukoc. Biol., 104: 253-264 (2018)
[Non Patent Literature 4]
   Fabbi, M. et al., J. Leukoc. Biol., 97: 665-675 (2015)
[Non Patent Literature 5]
   Zhou, T. et al., Nature, 583(7817): 609-614 (2020)
[Non Patent Literature 6]
   Dong, W. et al., Cancer Discov., 9(10): 1422-1437 (2019)
[Non Patent Literature 7]
   Senju, H. et al., Int. J. Biol. Sci., 14(3): 331-340 (2018)
[Non Patent Literature 8]
   Rahimi Kalateh Shah, M.G. et al., J. Cell. Physiol., 235(7-8): 5449-5460 (2020)

### [Summary of Invention]

### [Technical Problem]

The present invention aims to provide novel and effective cancer immunotherapeutic agents that exhibit therapeutic effects on intractable cancers, particularly cancers for which immune checkpoint inhibitor(s) monotherapy or combined use thereof with other agent(s) is ineffective.

### [Solution to Problem]

Spontaneous mice are used as a model for liver cancer that is difficult to treat, but there is almost no effective therapeutic drug for this model, and even lenvatinib, which is the standard drug for primary treatment of progressive hepatocellular cancer, is ineffective. The present inventors used a spontaneous hepatocellular cancer model mouse (Mdr2 KO mouse) in which the mdr2 gene encoding P-glycoprotein was deleted, and investigated the therapeutic effects of combined administration of an immune checkpoint inhibitor (anti-PD-L1 antibody), which has been shown to be effective in a peritoneal seeding model by colon cancer cell transfer and a lung metastasis model by melanoma cell transfer, and IL-18. As a result, a tendency toward increase in the tumor size was found, suggesting that the combination of an immune checkpoint inhibitor and IL-18 is not sufficiently effective for intractable liver cancer.

Therefore, the present inventors conducted intensive studies and found that combined administration of the above-mentioned two agents and a T-cell growth factor (e.g., IL-2) to Mdr2 KO mice results in a tendency of decreasing tumor size. Combined administration of two agents of an immune checkpoint inhibitor and IL-2 showed a tendency of increasing tumor size and a sufficient therapeutic effect was not obtained. The serum level of the tumor marker α-fetoprotein (AFP) significantly decreased by the combination of three agents compared with any two-agent combination. In addition, the production of antitumor cytokines (interferon-γ (IFN-γ) and tumor necrosis factor-α (TNF-α)) significantly increased by the combination of three agents, showing an increasing tendency as compared with any two-agent combination.

The effects of tumor shrinkage, decrease in AFP value, and increase in IFN-γ/TNF-α production by the three-agent combination became ineffective by the administration of anti-CD8 antibody. Therefore, at least CD8-positive T cell was considered to be important for the therapeutic effects by three-agent combination. The effects of tumor shrinkage by the three-agent combination became ineffective by the administration of anti-asialo-GM1 antibody. Therefore, the involvement of NK cells in the therapeutic effects by three-agent combination was also suggested.

Investigation of the expression of IL-18BP in Mdr2 KO mice, which is suggested to be a barrier to cancer treatment with IL-18, revealed that the protein level of IL-18BP in the serum did not change by two-agent combination of anti-PD-L1 antibody and IL-18, increased by two-agent combination of anti-PD-L1 antibody and IL-2, and significantly decreased by three-agent combination. Investigation of the IL-18BP protein level in the serum of hepatocellular cancer patients and intrahepatic bile duct cancer patients revealed that the IL-18BP level markedly increased in the hepatocellular cancer patients as compared with that of healthy subjects. However, since differences of up to 4-fold were observed between patients, it was suggested that a three-agent combination therapy may be effective in cancer patients with elevated serum levels of IL-18BP.

The present inventors have conducted further studies based on these findings and completed the present invention.

Accordingly, the present invention is defined in the claims and provides the following.

### [item 1]

A combination of an immune checkpoint inhibitor which is an anti-PD-L1 antibody or an anti-PD-1 antibody, interleukin-18 (IL-18), and a T cell growth factor which is IL-2, for use in a method of treating a liver cancer resistant to monotherapy with an immune checkpoint inhibitor or a combination therapy with an immune checkpoint inhibitor and IL-18.

### [item 2]

The combination of the immune checkpoint inhibitor, IL-18, and the T cell growth factor for use according to [item 1], which is administered to a subject with a serum IL-18 binding protein (IL-18BP) level of not less than 10 pg/mL.

### [Advantageous Effects of Invention]

A combination cancer immunotherapeutic agent is provided that is effective for intractable cancers such as liver cancer for which monotherapy with an immune checkpoint inhibitor or combined use thereof with IL-18 is ineffective.

### [Brief Description of Drawings]

[Fig. 1]
   A diagram schematically showing the drug administration and test schedule for Mdr2 KO mice.
[Fig. 2-1]
   Representative example of CT images showing tumor size at the start of administration (Baseline) and 4 weeks after the start of administration (4W) of Mdr2 KO mice in the control (PBS administration) group. The lower right is a photograph of the liver that was excised 4 weeks after the start of administration.
[Fig. 2-2]
   Representative example of hematoxylin eosin (HE) stained images of the liver of Mdr2 KO mice in the control (PBS administration) group 4 weeks after the start of administration.
[Fig. 3-1]
   Representative example of CT images showing reduction in tumor size in Mdr2 KO mice by administration of three agents (anti-PD-L1 antibody, IL-18, and IL-2). Baseline: at the start of administration, 4W: 4 weeks after the start of administration. The lower right is a photograph of the liver that was excised 4 weeks after the start of administration.
[Fig. 3-2]
   Representative example of HE stained images of the liver 4 weeks after the start of administration in the three agents (anti-PD-L1 antibody, IL-18, and IL-2) administration group.
[Fig. 4]
   Representative example of CT images showing an increase in tumor size in Mdr2 KO mice in two agents (anti-PD-L1 antibody and IL-2) administration group. Baseline: at the start of administration, 4W: 4 weeks after the start of administration. The lower right is a photograph of the liver that was excised 4 weeks after the start of administration.
[Fig. 5]
   Representative example of CT images showing an increase in tumor size in Mdr2 KO mice in two agents (anti-PD-L1 antibody and IL-18) administration group. Baseline: at the start of administration, 4W: 4 weeks after the start of administration.
[Fig. 6]
   (A) A diagram showing serum AFP levels (ng/mL) of Mdr2 KO mice in each administration group 4 weeks after the start of administration. (B) A diagram showing serum AFP levels (ng/mL) of Mdr2 KO mice of the three agents (anti-PD-L1 antibody, IL-18, and IL-2) administration group and the control (PBS administration) group at the start of administration (Baseline) and 4 weeks after the start of administration (4W).
[Fig. 7]
   Diagrams showing serum IFN-γ and TNF-α levels (ng/mL) of Mdr2 KO mice in each administration group 4 weeks after the start of administration, and serum IFN-γ and TNF-α levels (ng/mL) of Mdr2 KO mice of the three agents (anti-PD-L1 antibody, IL-18, and IL-2) administration group and the control (PBS administration) group at the start of administration (Baseline) and 4 weeks after the start of administration (4W).
[Fig. 8]
   Representative example of CT images showing that the tumor reduction effect in Mdr2 KO mice by administration of three agents (anti-PD-L1 antibody, IL-18, and IL-2) becomes ineffective by removal of NK cells. Baseline: at the start of administration, 4W: 4 weeks after the start of administration. The lower right is a photograph of the liver that was excised 4 weeks after the start of administration.
[Fig. 9-1]
   Representative example of CT images showing that the tumor reduction effect in Mdr2 KO mice by administration of three agents (anti-PD-L1 antibody, IL-18, and IL-2) is eliminated by removal of CD8-positive T cells. Baseline: at the start of administration, 4W: 4 weeks after the start of administration. The lower right is a photograph of the liver that was excised 4 weeks after the start of administration.
[Fig. 9-2]
   Diagrams showing that serum AFP level reducing action and production promoting action of IFN-γ and TNF-α levels in Mdr2 KO mice by administration of three agents (anti-PD-L1 antibody, IL-18 and IL-2) are eliminated by removal of CD8-positive T cells.
[Fig. 10-1]
   Diagrams showing serum IL-18BP level (pg/mg) of Mdr2 KO mice in each administration group 4 weeks after the start of administration, and serum IL-18BP level (pg/mg) of Mdr2 KO mice of the three agents (anti-PD-L1 antibody, IL-18, and IL-2) administration group and the control (PBS administration) group at the start of administration (Baseline) and 4 weeks after the start of administration (4W).
[Fig. 10-2]
   A diagram showing that serum IL-18BP level reducing action in Mdr2 KO mice by three agents (anti-PD-L1 antibody, IL-18 and IL-2) administration is eliminated by removal of CD8-positive T cells.
[Fig. 11-1]
   A diagram showing serum IL-18BP levels (pg/mL) in hepatocellular cancer patients (HCC), intrahepatic bile duct cancer patients (CCC), and healthy volunteers.
[Fig. 11-2]
   A diagram showing serum IL-18BP levels (pg/mL) in hepatitis C virus (HCV)-positive hepatocellular cancer patients (HCC), chronic hepatitis patients (CH), cirrhosis patients (LC), and healthy volunteers.
[Fig. 11-3]
   A diagram showing the examination results of the correlation between IL-18 and IL-18BP levels in the serum of hepatitis C virus (HCV)-positive hepatocellular cancer patients.

### [Description of Embodiments]

The present disclosure provides cancer therapeutic agents containing an immune checkpoint inhibitor which is an anti-PD-L1 antibody or an anti-PD-1 antibody, IL-18, and a T cell growth factor which is IL-2 in combination (hereinafter also to be referred to as "the combination agent of the present invention"). That is, the combination agent of the present invention is a combination cancer immunotherapeutic agent containing (a) an immune checkpoint inhibitor which is an anti-PD-L1 antibody or an anti-PD-1 antibody, (b) IL-18, and (c) a T cell growth factor which is IL-2 as active ingredients.

### (I) active ingredients

### (a) immune checkpoint inhibitor

In the present specification, the "immune checkpoint inhibitor" means a drug that inhibits the binding of immunosuppressive co-stimulatory molecules (immune checkpoint molecules) expressed on T cells or NK cells, and ligand thereof expressed on cancer cells and antigen-presenting cells, blocks the transmission of immunosuppressive signals, and releases suppression of the activation of T cells or NK cells.

An immune checkpoint inhibitor used as the active ingredient of the combination agent of the present invention is an anti-PD-L1 antibody or an anti-PD-1 antibody

Antibodies against immune checkpoint molecules or ligands thereof may be either polyclonal antibodies or monoclonal antibodies, but are preferably monoclonal antibodies. These antibodies can be produced according to production methods of antibody or antiserum known per se. The isotype of the antibody is not particularly limited, but IgG, IgM, or IgA is preferred, and IgG is particularly preferred. Since the Fc region of IgG1 and IgG2 has effector functions such as ADCC, ADCP, and CDC, it can be preferably used in antibodies targeting ligands expressed in cancer cells. On the other hand, since IgG4 has low effector function, it can be preferably used in antibodies targeting immune checkpoint molecules expressed on T cells and NK cells. Note that CTLA4 is also strongly expressed in regulatory T cells (Treg), and IgG1 or IgG2 subtypes can also be used as anti-CTLA4 antibodies in expectation of Treg removing action.

An antibody against an immune checkpoint molecule or a ligand thereof is not particularly limited as long as it has at least a complementarity determining region (CDR) for specifically recognizing and binding a target antigen, and may be a complete antibody molecule, as well as, for example, fragments such as Fab, Fab', F(ab')₂, genetically engineered conjugate molecule such as scFv, scFv-Fc, minibody, diabody or derivative thereof modified with polyethylene glycol (PEG) and the like having a protein stabilizing action.

Since antibodies to immune checkpoint molecules or ligands thereof are used as pharmaceutical products for human as the subject of administration, the antibodies (preferably monoclonal antibodies) may be antibodies with a reduced risk of exhibiting antigenicity when administered to humans, specifically, fully human antibody, humanized antibody, mouse-human chimeric antibody and the like, particularly preferably fully human antibody. Humanized antibodies and chimeric antibodies can be produced by genetic engineering according to conventional methods. In addition, fully human antibodies can also be produced from human-human (or mouse) hybridomas, but in order to stably provide large amounts of antibodies at low cost, human antibody-producing mice and phage display methods are desirably used for the production.

Some monoclonal antibodies against immune checkpoint molecules or ligands thereof are already on the market as pharmaceutical products or are in clinical trials and can be used. For example, anti-PD-1 antibodies include nivolumab (OPDIVO (registered trademark)), pembrolizumab (KEYTRUDA (registered trademark)), AMP-514 (MEDI0680), visilizumab (CT-011), and the like, anti-PD-L1 antibodies include atezolizumab (RG7446, MPDL3280A), durvalumab (MEDI4736), avelumab (PF-06834635, MSB0010718C), BMS-936559 (MDX1105) and the like. For reference, anti-CTLA4 antibodies include ipirimumab (YERVOY (registered trademark)), tremelimumab, and the like, anti-TIM-3 antibodies include MBG453 and the like, anti-LAG-3 antibodies include BMS-986016, LAG525, and the like, and anti-KIR antibodies include lirilumab and the like.

A substance that binds to immune checkpoint molecules to inhibit the binding thereof to ligands thereof expressed on antigen-presenting cells, such as cancer cells and tumor-infiltrating macrophages, may be a fragment that includes a portion (e.g., extracellular domain) necessary for binding of the ligand to an immune checkpoint molecule and does not have the ability to transmit an immunosuppressive signal, and further, a substance in which the fragment is conjugated with a molecule capable of removing immune exhausted- effector cells. Examples of the latter include fusion proteins (e.g., AMP-224, etc.) between the extracellular domain of PD-L1 or PD-L2 and the Fc region of an IgG1 or IgG2 antibody.

A substance that binds to immune checkpoint molecules to inhibit the binding thereof to ligands thereof may also be an antagonist that binds to the immune checkpoint molecule competitively with the ligand. Such antagonists can be obtained by constructing a competitive assay system using an immune checkpoint molecule and ligand thereof and screening a compound library.

In a combination agent, any one kind of the above-mentioned immune checkpoint inhibitors can be used or two or more kinds thereof can be used in combination.

### (b)IL-18

IL-18 is a pro-inflammatory cytokine belonging to the IL-1 family and promotes IFN-γ production by T cells and NK cells. Human IL-18 is produced as an inactive precursor (pro-IL-18) consisting of 192 amino acids. However, when a protein complex called inflammasome is activated by PAMPs or DAMPs, caspase-1 is activated, and the enzymatic activity thereof processes pro-IL-18, and an active mature IL-18 consisting of 157 amino acids is generated. For the amino acid sequence information of human IL-18, for example, UniProtKB (accession number:Q14116) can be referred to. The amino acid sequence of human mature IL-18 is shown in SEQ ID NO: 2.

IL-18 used as the active ingredient of the combination agent of the present invention is a protein comprising the same or substantially the same amino acid sequence as the amino acid sequence shown in SEQ ID NO:2. IL-18 may be, for example, a protein isolated and purified from IL-18-producing cells (e.g., macrophage, dendritic cell, microglia, synovium fibroblast, epithelial cell, etc.) or tissues containing them from humans and other mammals (e.g., rat, mouse, monkey, dog, bovine, rabbit, swine, sheep, and the like). It may also be a chemically-synthesized protein or a protein biochemically synthesized in a cell-free translation system. Alternatively, the protein may be a recombinant protein produced from a transformant incorporating a nucleic acid having a base sequence that encodes the above-described amino acid sequence.

Substantially the same amino acid sequence as the amino acid sequence shown in SEQ ID NO:2 refers to an amino acid sequence having an identity of 80% or more, preferably 90% or more, more preferably 95% or more, particularly preferably 97% or more, most preferably 98% or more, to the amino acid sequence shown in SEQ ID NO:2, and the like. Here, the "identity" means a ratio (%) of identical amino acid residues to all overlapping amino acid residues in the optimal alignment (preferably, the algorithm considers introduction of gaps on one or both sides of the sequence for the best alignment) where two amino acid sequences are aligned using a mathematical algorithm known in the technical field. The identity of the amino acid sequence in the present specification can be calculated using the homology calculation algorithm NCBI BLAST (National Center for Biotechnology Information Basic Local Alignment Search Tool) under the following conditions (expectancy=10; gaps allowed; matrix=BLOSUM62; filtering=OFF).

IL-18 is a protein having substantially the same amino acid sequence as the amino acid sequence shown in SEQ ID NO:2, and having an activity of the same quality as that of a protein containing the amino acid sequence shown in SEQ ID NO:2. Here, the "activity" refers to, for example, any activity that contributes to the suppression of cancer such as binding activity with receptors, and activity promoting the proliferation and activation of T cells and NK cells. Being "of the same quality" means that the activities thereof are qualitatively the same. Therefore, it is preferable that the activity of IL-18 be equivalent to or not less than that of wild-type IL-18, but the extent of the activities of these may be different.

Examples of the IL-18 used in the present invention also include proteins including (i) an amino acid sequence resulting from deletion of one or two or more (e.g., about 1 to 30, preferably about 1 to 10, further preferably 1 to several (5, 4, 3, or 2)) amino acids in the amino acid sequence shown in SEQ ID NO: 2, (ii) an amino acid sequence resulting from addition of one or two or more (e.g., about 1 to 30, preferably about 1 to 10, further preferably 1 to several (5, 4, 3, or 2)) amino acids to the amino acid sequence shown in SEQ ID NO: 2, (iii) an amino acid sequence resulting from insertion of one or two or more (e.g., about 1 to 30, preferably about 1 to 10, further preferably 1 to several (5, 4, 3, or 2)) amino acids into the amino acid sequence shown in SEQ ID NO: 2, (iv) an amino acid sequence resulting from substitution of one or two or more (e.g., about 1 to 30, more preferably about 1 to 10, further preferably 1 to several (5, 4, 3, or 2)) amino acids with other amino acids in the amino acid sequence shown in SEQ ID NO: 2, or (v) an amino acid sequence combining them, and the like.

When an amino acid sequence has insertion, deletion, or substitution as described above, the position of the insertion, deletion, or substitution is not particularly limited. Examples of the IL-18 variant include a variant resulting from substitution of at least one of the 38-position, the 68-position, the 76-position, and the 127-position cysteine (Cys) residues with other amino acid (e.g., Ser, Ala, Asp, Thr, Val, Leu, etc.) in the amino acid sequence shown in SEQ ID NO: 2 and having improved stability (e.g., JP-B-4024366), a variant having amino acid substitution of L144C or D157C in addition to C38S, C68S, or C68D, and having altered affinity for IL-18 receptor and/or IL-18BP (e.g., JP-B-4753867), a variant wherein an amino acid residue important for the binding with IL-18BP, for example, at least one amino acid residue from the 42-position Glu, the 85-position Ile, the 87-position Met, the 89-position Lys, the 96-position Met, the 130-position Asp, the 132-position Lys, the 143-position Pro, the 149-position Met, and the 189-position Leu, preferably G42 and/or K89, in the amino acid sequence shown in SEQ ID NO: 2 is substituted with other amino acid, and having decreased affinity for IL-18BP (e.g., JP-A-2004-530432), and an IL-18BP low affinity variant with substitution of at least one of Y1X, L5X, K8X, M51X, K53X, S55X, Q56X, P57X, G59X, M60X, E77X, Q103X, S105X, D110X, N111X, M113X, V153X, and N155X (X is an amino acid different from the amino acid residues of wild-type IL-18), preferably M51X, M60X, S105X, D110X, and N111X, or M51X, K53X, Q56X, S105X, and N111X (e.g., JP-A-2020-533301). Alternatively, the IL-18 variants (i.e., C38/68/76/127S-E6A-K53A, C68/76/127S-E6A-K53A-C38M, C38/68/76/127S-E6A-K53A-G3Y, C38/68/76/127S-E6A-K53A-G3L, C38/68/76/127S-E6A-K53A-S72Y, C38/68/76/127S-E6A-K53A-S72M, C38/68/76/127S-E6A-K53A-S72F) created by the present inventors can also be used preferably.

The IL-18 used in the present invention may be a free form or a salt. As such salt, salts with physiologically acceptable acid (e.g., inorganic acid, organic acid), base (e.g., alkali metal, alkaline earth metal) and the like are used, and a physiologically acceptable acid addition salt is particularly preferred. Examples of such salt to be used include salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

IL-18 can be isolated from extracellular matrices and culture supernatants of the aforementioned IL-18-producing cells or tissues of human and other mammals, by a protein purification method known per se, for example, chromatography such as reversed-phase chromatography, ion exchange chromatography, affinity chromatography and the like. IL-18 can also be produced according to a known method of peptide synthesis. The method of peptide synthesis may be any of, for example, a solid phase synthesis process and a liquid phase synthesis process. That is, it can be produced by condensing a partial peptide or amino acid capable of constituting IL-18 with the remaining portion, and removing any protecting group the resultant product may have.

IL-18 can be produced by culturing a transformant comprising a nucleic acid encoding the same, and separating and purifying IL-18 from the obtained culture. The nucleic acid here may be DNA or RNA. In the case of DNA, it is preferably a double-stranded DNA. DNA encoding IL-18 can be cloned by, for example, synthesizing oligo DNA primers based on the cDNA sequence information thereof, and amplifying by RT-PCR method by using, as a template, a total RNA or mRNA fraction prepared from IL-18-producing cells. Using the obtained cDNA as a template, the above-mentioned various mutations can be introduced using site-directed mutagenesis methods known per se.

Examples of the DNA encoding IL-18 include a DNA containing the base sequence shown in SEQ ID NO: 1, a DNA encoding a protein containing a base sequence that hybridizes with the base sequence shown in SEQ ID NO: 1 under stringent conditions and having an activity qualitatively the same as that of the aforementioned wild-type IL-18, and the like. As the DNA capable of hybridizing with the base sequence shown in SEQ ID NO: 1 under stringent conditions, a DNA containing a base sequence having an identity of 80% or more, preferably 90% or more, more preferably 95% or more, particularly preferably 97% or more, most preferably 98% or more, with the base sequence shown in SEQ ID NO: 1 or the like is used.

The identity of the base sequence in the present specification can be calculated using the homology calculation algorithm NCBI BLAST (National Center for Biotechnology Information Basic Local Alignment Search Tool) under the following conditions (expectancy=10; gap allowed; filtering=ON; match score=1; mismatch score=-3).

The DNA encoding IL-18 is preferably a DNA encoding wild-type human IL-18 having the base sequence shown in SEQ ID NO: 1, or a DNA encoding the aforementioned various human IL-18 variant proteins.

As the DNA encoding IL-18, a DNA encoding the full-length thereof can also be constructed by chemically synthesizing a DNA strand, or connecting a synthesized, partially overlapping oligo-DNA short strand by the PCR method or the Gibson assembly method. The advantage of constructing a full-length DNA by chemical synthesis or a combination with PCR method or Gibson Assembly method is that the codon to be used can be designed for the full length of CDS to suit the host into which the DNA is introduced. When expressing heterologous DNA, an increase in protein expression level can be expected by converting the DNA sequence to codons that are frequently used in the host organism. Data of codon usage frequency in the host used can be obtained from, for example, the genetic code usage frequency database (http://www.kazusa.or.jp/codon/index.html) published on the website of (PIIF) the Kazusa DNA Research Institute) or reference can be made to literature that describes codon usage frequency in each host.

Cloned DNA may be, depending on the object, directly used, or used after digestion with a restriction enzyme when desired, or addition of a linker. The DNA may have ATG as a translation initiation codon on the 5' terminal side, and may have TAA, TGA or TAG as a translation stop codon on the 3' terminal side. These translation initiation codon and translation stop codon can be added using a suitable synthesized DNA adapter.

An expression vector comprising DNA that encodes IL-18 can be produced by, for example, cutting out a desired DNA fragment from the DNA that encodes IL-18, and joining the DNA fragment downstream of a promoter in an appropriate expression vector. As the expression vector, plasmid derived from Escherichia coli (e.g., pBR322, pBR325, pUC12, pUC13); plasmid derived from Bacillus subtilis (e.g., pUB110, pTP5, pC194); plasmid derived from yeast (e.g., pSH19, pSH15); insect cell expression plasmid (e.g., pFast-Bac); animal cell expression plasmid (e.g., pA1-11, pXT1, pRc/CMV, pRc/RSV, pcDNAI/Neo); bacteriophage such as λphage and the like; insect virus vector (e.g., BmNPV, AcNPV) such as baculovirus and the like; animal virus vector such as retrovirus, lentivirus, vaccinia virus, adenovirus, adeno-associated virus, herpes virus, and the like, and the like are used.

The promoter may be any promoter, as long as it is appropriate for the host used to express the gene. For example, when the host is an animal cell, SRα promoter, SV40 promoter, LTR promoter, CMV (cytomegalovirus) promoter, RSV (Rous sarcoma virus) promoter, MoMuLV (Moloney murine leukemia virus) LTR, HSV-TK (simple herpes virus thymidine kinase) promoter and the like are used. Promoters known per se can be appropriately selected for other hosts.

As the expression vector, in addition to the above, those optionally harboring an enhancer, a splicing signal, a polyA addition signal, a selection marker, an SV40 replication origin (hereinafter also abbreviated as SV40 ori) and the like can be used. As examples of the selection marker, the dihydrofolate reductase gene, the ampicillin resistance gene, the neomycin resistance gene, and the like can be mentioned.

IL-18 can be produced by transforming a host with an expression vector containing the above-mentioned DNA encoding IL-18, and cultivating the obtained transformant. As the host, for example, genus Escherichia, genus Bacillus, yeast, insect cell, insect, animal cell, and the like are used. As the mammal cell, for example, monkey COS-7 cell, monkey Vero cell, Chinese hamster ovary cell (hereinafter to be abbreviated as CHO cell), dhfr gene deficient CHO cell (hereinafter to be abbreviated as CHO(dhfr⁻) cell), mouse L cell, mouse AtT-20 cell, mouse myeloma cell, rat GH3 cell, human FL cell, HeLa cell, HepG2 cell, HEK293 cell, and the like are used. Cells known per se can be appropriately selected for other hosts.

Transformation can be carried out according to the kind of host in accordance with a publicly known method. An animal cell can be transformed, for example, in accordance with a method described in Saibo Kogaku (Cell Engineering), extra issue 8, Shin Saibo Kogaku Jikken Protocol (New Cell Engineering Experimental Protocol), 263-267 (1995), published by Shujunsha, or Virology, Vol. 52, 456 (1973).

Cultivation of a transformant can be performed according to the kind of host in accordance with a publicly known method.

For example, as a medium for cultivating a transformant whose host is an animal cell, minimum essential medium (MEM), Dulbecco's modified Eagle medium (DMEM), RPMI 1640 medium, 199 medium, Ham's F-12 medium, and the like containing about 5 to about 20% fetal bovine serum are used. The medium's pH is preferably about 6 to about 8. Cultivation is normally performed at about 30°C to about 40°C for about 15 to about 60 hours. Where necessary, aeration or agitation may be performed.

As described above, IL-18 can be produced in a cell of the transformant or outside the cell.

IL-18 can be separated and purified from the culture obtained by cultivating the aforementioned transformant according to a method known per se. Such methods include methods based on solubility, such as salting-out and solvent precipitation; methods based mainly on molecular weight differences, such as dialysis, ultrafiltration, gel filtration, and SDS-polyacrylamide gel electrophoresis; methods based on charge differences, such as ion exchange chromatography; methods based on specific affinity, such as affinity chromatography; methods based on hydrophobicity differences, such as reversed-phase high performance liquid chromatography; and methods based on isoelectric point differences, such as isoelectric focusing. These methods can be combined as appropriate.

When the thus-obtained IL-18 is in a free form, the free form can be converted into a suitable salt form by a known method or a method analogous thereto, and when the IL-18 is obtained in the form of a salt, it can be converted into a free form or in the form of a different salt by a known method or a method analogous thereto.

### (c) T cell growth factor

The T cell growth factor used in the combination agent of the present invention is a humoral factor that causes the proliferation and activation of T cells including CTL and Th1 cells, and is IL-

2. Other cytokines mentioned herein include IL-15, IL-7, IL-9, IL-21, and the like. T T cell growth factor can also cause proliferation of NK cells (e.g., IL-2, IL-15). In this case, the T cell growth factor is sometimes referred to as "NK cell and T cell growth factor".

IL-2 is produced by activated T cells, NK cells, and dendritic cells and causes proliferation and activation of cells that exhibit tumoricidal action such as CTL and NK cells. Human IL-2 is produced as a precursor consisting of 153 amino acids, the N-terminal signal sequence consisting of 20 amino acids is cleaved and secreted as mature IL-2 consisting of 133 amino acids. For the amino acid sequence information of human IL-2, for example, UniProtKB (accession number:P60568) can be referred to. The amino acid sequence of human mature IL-2 is shown in SEQ ID NO: 4.

IL-15 is produced by monocytes, macrophages, dendritic cells, and the like and exhibits action similar to that of IL-2 on CTL and NK cells. Human IL-15 is produced as a precursor consisting of 162 amino acids, the N-terminal signal sequence consisting of 29 amino is produced as a precursor consisting of 162 amino acids, the N-terminal signal sequence consisting of 29 amino acids is cleaved and secreted as an inactive pro-form, and a pro-peptide consisting of 19 amino acids is further cleaved to form a mature IL-15 (active form) consisting of 114 amino acids. For the amino acid sequence information of human IL-15, for example, UniProtKB (accession number:P40933) can be referred to. The amino acid sequence of human mature IL-15 is shown in SEQ ID NO: 6.

IL-7 is produced by interstitial cells, fibroblastic reticulum cells, thymus epithelial cells, and the like and contributes to the promotion of initial differentiation of T cells, quantitative maintenance of peripheral T cells, and the like. Human IL-7 is produced as a precursor consisting of 177 amino acids, the N-terminal signal sequence consisting of 25 amino acids is cleaved and secreted as mature IL-7 consisting of 152 amino acids. For the amino acid sequence information of human IL-7, for example, UniProtKB (accession number:P13232) can be referred to. The amino acid sequence of human mature IL-7 is shown in SEQ ID NO: 8.

IL-2, IL-15, and IL-7, are respectively each a protein having the same or substantially the same amino acid sequence as each amino acid sequence shown in SEQ ID NO: 4, 6, and 8. IL-2, IL-15, and IL-7 may be proteins isolated and purified from cytokine-producing cells (e.g., T cell, NK cell, NKT cell, activated dendritic cell, mast cell, and the like as IL-2-producing cells, monocyte, macrophage, dendritic cell, and the like as IL-15-producing cells, interstitial cell, fibroblastic reticulum cell, thymus epithelial cell, and the like as IL-7-producing cells) or tissues containing them from humans and other mammals (e.g., rat, mouse, monkey, dog, bovine, rabbit, swine, sheep, and the like). They may also be chemically-synthesized proteins or proteins biochemically synthesized in a cell-free translation system. Alternatively, the proteins may be recombinant proteins produced from a transformant incorporating a nucleic acid having a base sequence that encodes each of the above-described amino acid sequences.

Substantially the same amino acid sequence as the amino acid sequence shown in SEQ ID NO: 4, 6, or 8 refers to an amino acid sequence having an identity of 80% or more, preferably 90% or more, more preferably 95% or more, particularly preferably 97% or more, most preferably 98% or more, to the amino acid sequence shown in SEQ ID NO: 4, 6, or 8, and the like. Here, the "identity" is as described above for IL-18.

IL-2, IL-15, and IL-7 are respectively each a protein having substantially the same amino acid sequence as each amino acid sequence shown in SEQ ID NO: 4, 6, and 8, and having an activity of the same quality as that of a protein containing each amino acid sequence shown in SEQ ID NO: 4, 6, and 8. Here, the "activity" refers to, for example, any activity that contributes to the suppression of cancer such as binding activity with receptors, and activity promoting the differentiation, proliferation, and activation of T cells and NK cells. Being "of the same quality" means that the activities thereof are qualitatively the same. Therefore, it is preferable that the activities of IL-2, IL-15, and IL-7 be equivalent to or not less than those of wild-type IL-2, wild-type IL-15 and wild-type IL-7, but the extent of the activities of these may be different.

Alternatively, the IL-2, IL-15, and IL-7 may also include proteins including (i) an amino acid sequence resulting from deletion of one or two or more (e.g., about 1 to 30, preferably about 1 to 10, further preferably 1 to several (5, 4, 3, or 2)) amino acids in the amino acid sequence shown in SEQ ID NO: 4, 6, or 8, (ii) an amino acid sequence resulting from addition of one or two or more (e.g., about 1 to 30, preferably about 1 to 10, further preferably 1 to several (5, 4, 3, or 2)) amino acids to the amino acid sequence shown in SEQ ID NO: 4, 6, or 8, (iii) an amino acid sequence resulting from insertion of one or two or more (e.g., about 1 to 30, preferably about 1 to 10, further preferably 1 to several (5, 4, 3, or 2)) amino acids into the amino acid sequence shown in SEQ ID NO: 4, 6, or 8, (iv) an amino acid sequence resulting from substitution of one or two or more (e.g., about 1 to 30, more preferably about 1 to 10, further preferably 1 to several (5, 4, 3, or 2)) amino acids with other amino acids in the amino acid sequence shown in SEQ ID NO: 4, 6, or 8, or (v) an amino acid sequence combining them, and the like.

When an amino acid sequence has insertion, deletion, or substitution as described above, the position of the insertion, deletion, or substitution is not particularly limited. As the IL-2 variant, a variant resulting from substitution of at least one of the 3-position Thr, the 42-position Phe, the 45-position Tyr, and the 72-position Leu residues with other amino acid (e.g., Ala, Ser, Val, etc.) in the amino acid sequence shown in SEQ ID NO: 4, and having decreased affinity for the IL-2Rα chain of the high-affinity αβγc receptor expressed on Tregs (e.g., JP-B-2020-33362), and the like can be mentioned. Variants known per se and having an activity equal to or higher than that of the wild type can be used also for IL-15, IL-7.

The IL-2, IL-15, and IL-7 may be a free form or a salt. As such salt, salts similar to those described above for IL-18 can be recited.

IL-2, IL-15, and IL-7 can be isolated from extracellular matrices and culture supernatants of the aforementioned cytokine-producing cells or tissues of human and other mammals, by protein purification methods known per se. IL-2, IL-15, and IL-7 can also be produced according to a known method of peptide synthesis. As such protein purification method and peptide synthesis method, methods similar to those described above for IL-18 can be used.

IL-2, IL-15, and IL-7 can be produced by culturing transformants comprising nucleic acids encoding them, and separating and purifying them from the obtained cultures.

Examples of the DNA encoding IL-2, IL-15, or IL-7 include each a DNA containing the base sequence shown in SEQ ID NO: 3, 5, or 7, a DNA encoding a protein containing a base sequence that hybridizes with the base sequence shown in SEQ ID NO: 3, 5, or 7 under stringent conditions and having an activity qualitatively the same as that of the aforementioned wild-type IL-2, IL-15, or IL-7, and the like. As the DNA capable of hybridizing with the base sequence shown in SEQ ID NO: 3, 5, or 7 under stringent conditions, a DNA containing a base sequence having an identity of 80% or more, preferably 90% or more, more preferably 95% or more, particularly preferably 97% or more, most preferably 98% or more, with the base sequence shown in SEQ ID NO: 3, 5, or 7 or the like is used.

The identity of the base sequence in the present specification can be calculated in the same manner as in the case of DNA encoding IL-18.

The DNA encoding IL-2, IL-15, or IL-7 is preferably a DNA encoding wild-type human IL-2, IL-15, or IL-7 having the base sequence shown in SEQ ID NO: 3, 5, or 7, or a DNA encoding the aforementioned various human IL-2, IL-15, and IL-7 variant proteins.

As the DNA encoding IL-2, IL-15, and IL-7, a DNA encoding the full-length thereof can also be constructed by chemically synthesizing a DNA strand, or connecting a synthesized, partially overlapping oligo-DNA short strand by the PCR method or the Gibson assembly method.

Cloned DNA may be, depending on the object, directly used, or used after digestion with a restriction enzyme when desired, or addition of a linker. The DNA may have ATG as a translation initiation codon on the 5' terminal side, and may have TAA, TGA or TAG as a translation stop codon on the 3' terminal side. These translation initiation codon and translation stop codon can be added using a suitable synthesized DNA adapter.

An expression vector comprising DNA that encodes IL-2, IL-15, or IL-7 can be produced by, for example, cutting out a desired DNA fragment from the DNA that encodes IL-2, IL-15, or IL-7, and joining the DNA fragment downstream of a promoter in an appropriate expression vector. As the expression vector and promoter, those mentioned above with regard to IL-18 are preferably used in the same manner. The expression vector may contain enhancer, splicing signal, polyA addition signal, selection marker, SV40 ori, and the like when desired. As examples of the selection marker, the dihydrofolate reductase gene, the ampicillin resistance gene, the neomycin resistance gene, and the like can be mentioned.

IL-2, IL-15, or IL-7 can be produced by transforming a host with an expression vector containing the above-mentioned DNA encoding IL-2, IL-15, or IL-7, and cultivating the obtained transformant. As the host, transformation method, method for culturing transformant, method for purifying the obtained IL-2 protein, method for converting a free form into a salt, or a salt into a free form, and the like, those or the methods described above for IL-18 can also be used preferably.

While IL-2, Il-15, and IL-7 have been described as examples of T cell growth factors, those skilled in the art can easily obtain other T cell growth factors based on the description in the present specification.

### (II) Combination agent of the present invention

As shown in the below-mentioned Example, by using three agents of immune checkpoint inhibitor, IL-18, and T cell growth factor in combination, tumor growth can be remarkably suppressed in naturally developed cancer model mice that serve as a model for intractable cancer where almost no drug is available that affords a therapeutic effect, as compared with combinations of immune checkpoint inhibitor and IL-18, and combinations of immune checkpoint inhibitor and T cell growth factor. Therefore, the combination agent of the present invention can be used as a therapeutic agent for liver cancer resistant to monotherapy with an immune checkpoint inhibitor or a combination therapy with an immune checkpoint inhibitor and IL-18. The "cancer treatment" here is used as a concept that encompasses all of suppressing tumor growth, progression, infiltration and metastasis of cancer, delaying cancer onset, and suppression of recurrence.

The type of cancer for which the combination agent of the present invention is used is liver cancer. Cancer may be derived from epithelial cells, or may be non-epithelial sarcoma or hematological cancer. Examples of cancer include, but are not limited to, gastrointestinal cancer (e.g., liver cancer (hepatocellular cancer, cholangiocellular carcinoma), gall bladder cancer, bile duct cancer, pancreatic cancer, duodenal cancer, esophageal cancer, gastric cancer, colorectal cancer (colon cancer, rectal cancer), anal cancer), urinary cancer (e.g., kidney cancer, ureteral cancer, bladder cancer, prostate cancer, penile cancer, testis (orchis) cancer), thorax cancer (e.g., breast cancer, lung cancer (non-small cell lung cancer, small cell lung cancer)), reproductive organ cancer (e.g., cancer of uterus (cervical cancer, uterine cancer), ovarian cancer, vulvar cancer, vaginal cancer), head and neck cancer (e.g., maxilla cancer, pharyngeal cancer, laryngeal cancer, tongue cancer, thyroid cancer), and skin cancer (e.g., basal cell cancer, spinous cell carcinoma).

The Mdr2 KO mice used in the Examples below develop chronic peribiliary inflammation and cholestatic liver disease due to a deficiency in the iAbc4 protein, which is a member of the ATP-binding cassette (ABC) transporter superfamily and is encoded by the mdr2 gene, and spontaneously develop hepatocellular cancer (Katzenellengoben et al., Mol. Cancer Res. 2007, 5(11): 1159-1170). Therefore, the cancer targeted by the combination agent of the present invention may be liver cancer derived from inflammation, fibrosis, or liver cirrhosis caused by viral infection with hepatitis B and C viruses (including metastatic cancers wherein these are primary sources).

The immune checkpoint inhibitor, IL-18, and T cell growth factor are of lower toxicity, and can be administered as a liquid or as a pharmaceutical composition in a suitable dosage form to human or other mammals orally or parenterally (e.g., intravascular administration (intravenous administration, intraarterial administration, etc.), subcutaneous administration, intradermal administration, intraperitoneal administration, muscle injection, topical administration, and the like).

In the combination agent of the present invention, the three active ingredients may be formulated separately, or two or more of them may be formulated as a single agent.

As a composition for parenteral administration, for example, injection, suppository and the like are used, and the injection may include the dosage forms of intravenous injection, subcutaneous injection, intradermal injection, intramuscular injection, drip injection, and the like. Such injection can be prepared according to a known method. Injection can be prepared, for example, by dissolving, suspending or emulsifying one or more active ingredients in a sterile aqueous solution or an oily solution generally used for injection. As the aqueous solution for injection, for example, saline, isotonic solution containing glucose and other auxiliary agents, and the like are used, which may be used in combination with a suitable solubilizing agent, for example, alcohol (e.g. ethanol), polyalcohol (e.g., propylene glycol, polyethylene glycol), nonionic surfactant [e.g., polysorbate 80, HCO-50 (polyoxyethylene (50 mol) adduct of hydrogenated castor oil)] and the like. As the oily solution, sesame oil, soybean oil and the like are used and, as a solubilizing agent, benzyl benzoate, benzyl alcohol and the like may be used in combination. A prepared injection is preferably filled in a suitable ampoule. A suppository to be used for rectal administration may be prepared by mixing the active ingredient with a general suppository base.

Examples of the composition for oral administration include solid or liquid dosage forms, which are specifically tablet (including sugar-coated tablet, film-coated tablet), pill, granule, powder, capsule (including soft capsule), syrup, emulsion, suspension and the like. Such composition is produced by a known method, and may contain a carrier, a diluent or an excipient generally used in the pharmaceutical field. As the carrier and excipient for tablets, lactose, starch, saccharose, and magnesium stearate are used.

The above-mentioned parenteral or oral pharmaceutical composition is conveniently prepared into a dosage form in a dosage unit compatible with the dose of the active ingredient. Examples of such dosage form in the dosage unit include tablet, pill, capsule, injection (ampoule), and suppository. The immune checkpoint inhibitor is generally contained in 10 to 10000 mg, preferably 50 to 1000 mg, more preferably 100 to 1000 mg, per unit dosage form. IL-18 is generally contained in 1 to 100 mg, preferably 5 to 50 mg, per unit dosage form. IL-2 is generally contained in 10⁴ to 10⁶ unit, preferably 5×10⁴ to 5×10⁵ unit, per unit dosage form.

The dose of each active ingredient varies depending on the subject of administration, target disease, symptom, administration route and the like. For example, when it is used for the treatment of liver cancer in an adult, it is convenient to administer generally about 0.5 to 500 mg/kg body weight, preferably about 2.5 to 50 mg/kg body weight, more preferably about 5 to 50 mg/kg body weight, of an immune checkpoint inhibitor in a single dose, generally about 0.05 to 1 mg/kg body weight, preferably about 0.25 to 2.5 mg/kg body weight, of IL-18 in a single dose, and generally about 5×10² to 5×10⁴ unit/kg body weight, preferably about 2.5×10³ to 2.5×10⁴ unit/kg body weight, of IL-2 in a single dose, by intravenous or intraperitoneal administration about once a day to once a week, preferably about 2 - 3 times a week. For other parenteral administration and oral administration, amounts in accordance therewith can be administered. When the symptom is particularly severe, the amount may be increased according to the symptom.

The ratio of the active ingredients in the combination agent of the present invention is not particularly limited as long as the dose of each active ingredient is within the above-mentioned range. For example, when the dose of IL-18 is 1, the dose of the immune checkpoint inhibitor may be about 5 to 200, preferably about 10 to 50. In addition, the dose of the T cell growth factor, IL-2, may be about 0.1 to 1, preferably about 0.2 to 0.5, with the dose of IL-18 as 1, converting one unit as 0.5 ng.

In the combination agent of the present invention, when each active ingredient is provided in the form of a separate composition, or any two agents are provided in the form of a single composition, and the remaining one agent is provided in the form of a separate composition, each composition can be administered to a subject by the same or separate routes, at the same time or at staggered intervals.

IL-18BP shows increased expression in the cancer microenvironment, binds to IL-18 with high affinity, blocks the binding to IL-18 receptor on effector cells such as T cells, NK cells, and the like and signal transduction, and can function as a soluble immune checkpoint molecule that suppresses the proliferation and activation of effector cells. Furthermore, it has been reported that IL-18BP levels are markedly increased in the serum of cancer patients who have undergone cytokine therapy with IL-18. As shown in the Examples below, IL-18BP levels are increased in the serum of spontaneous liver cancer model mice. The two-agent combination of an immune checkpoint inhibitor and IL-18 or IL-2 cannot decrease the IL-18BP level, whereas the combination agent of the present invention which combines three agents, can markedly reduce IL-18BP levels. In addition, serum IL-18BP levels in hepatocellular cancer patients who have not received IL-18 therapy are markedly higher than those in healthy subjects, and it was clarified that numerical values thereof vary greatly among patients. High serum IL-18BP levels are considered to reflect the establishment of an immunosuppressive state due to IL-18BP in the cancer microenvironment. Therefore, the combination agent of the present invention that can reduce the expression of IL-18BP in cancer patients with particularly high serum IL-18BP levels can be particularly useful because it can relieve immunosuppression (immune exhaustion of T cell, NK cell, and the like) by IL-18 in the patients. In fact, in the Examples described below, in spontaneous liver cancer model mice, although the number of individuals was small, the IL-18BP level remarkably decreased after administration of the three agents in individuals that showed particularly high serum IL-18BP levels at the start of the administration. This strongly suggests that subjects for whom the combination agent of the present invention is markedly effective can be identified using the serum IL-18BP level as an index.

IL-18BP is constitutively expressed even in healthy individuals, and normal serum level is said to be around several pg/mL. In one preferable embodiment, therefore, the combination agent of the present invention can be administered to cancer patients with a serum IL-18BP level of not less than 10 pg/mL, preferably not less than 20 pg/mL. The serum IL-18BP level can be measured, for example, by immunochemical assays (e.g., ELISA, etc.) using anti-IL-18BP antibody, and such ELISA kits are commercially available.

The present invention is explained in detail in the following by referring to Examples. The present invention is not limited to them.

### [Example]

### Example 1 Combination therapy of immune checkpoint inhibitor and IL-18 and/or IL-2 for Mdr2^{-/-} KO mouse

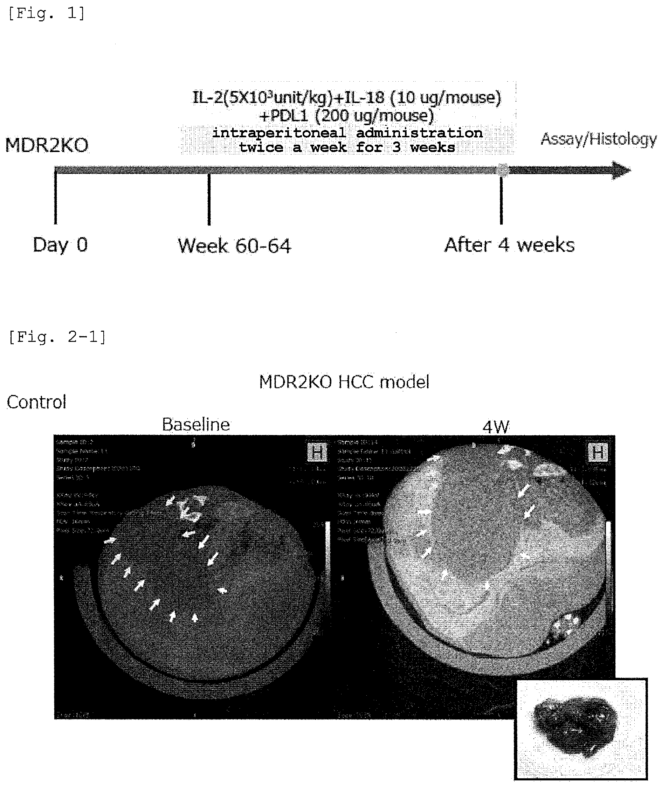

Mdr2^{-/-}KO mice (60- to 64-week-old, female; obtained from The Jackson Laboratory) that spontaneously developed hepatocellular cancer were divided into 4 groups and
1) anti-mouse PD-L1 antibody (clone 10F.9G2; purchased from BioXcell) 200 µg/mouse, IL-18 (recombinant mouse IL-18; manufactured by GlaxoSmithKline) 10 µg/mouse, and IL-2 (gene recombinant type IL-2 (IMUNACE35 for Injection); manufactured by Shionogi & Co., Ltd.) 5,000 unit/kg (IL-2+IL-18+PD-L1 administration group);
2) anti-mouse PD-L1 antibody 200 µg/mouse, and IL-2 5,000 unit/kg (IL-2+PD-L1 administration group);
3) anti-mouse PD-L1 antibody 200 µg/mouse, and IL-18 10 µg/mouse (IL-18+PD-L1 administration group); or
4) saline (Control (PBS administration) group); were intraperitoneally administered for 4 weeks at intervals of twice a week. Four weeks after the start of drug administration, the effects of each combination agent were examined. The schedule for this experiment is shown in Fig. 1.

### (1) tumor growth suppressive effect

Using a CT scan (experiment veterinary 3D micro X-RAY CT device (R_mCT2-SP2): manufactured by Rigaku Corporation), CT images of the liver were taken at the start of drug administration and 4 weeks after the start of drug administration. As a result, a tumor size-increasing tendency was observed in the Control group (Fig. 2-1), whereas a tumor size-decreasing tendency was observed in the IL-2+IL-18+PD-L1 administration group (Fig. 3 -1). On the other hand, a tumor size-increasing tendency was observed in the IL-2+PD-L1 administration group (Fig. 4) and the IL-18+PD-L1 administration group (Fig. 5), and it was clarified that two-agent combination of immune checkpoint inhibitor and one of the cytokines does not provide a tumor growth suppressive effect.

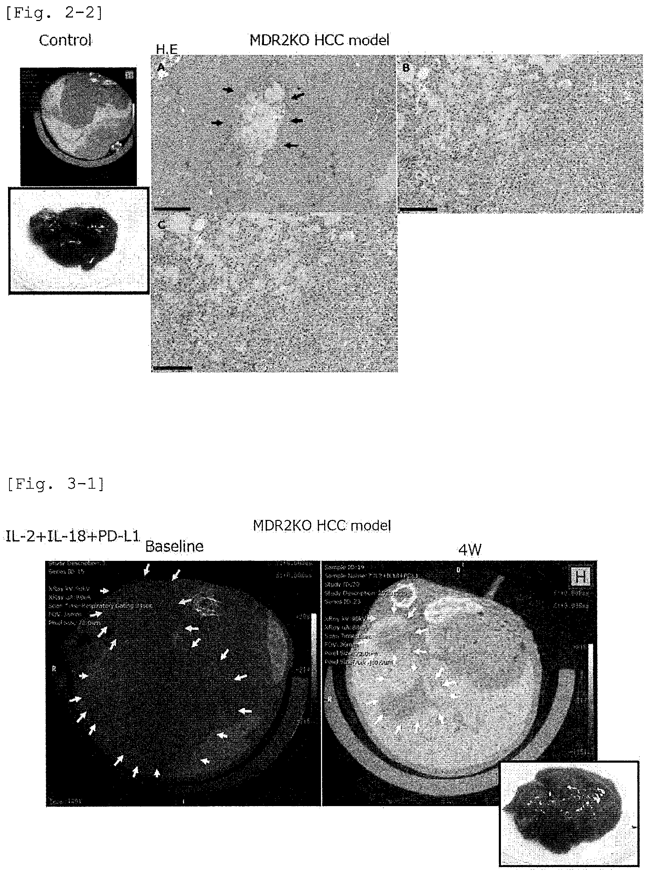

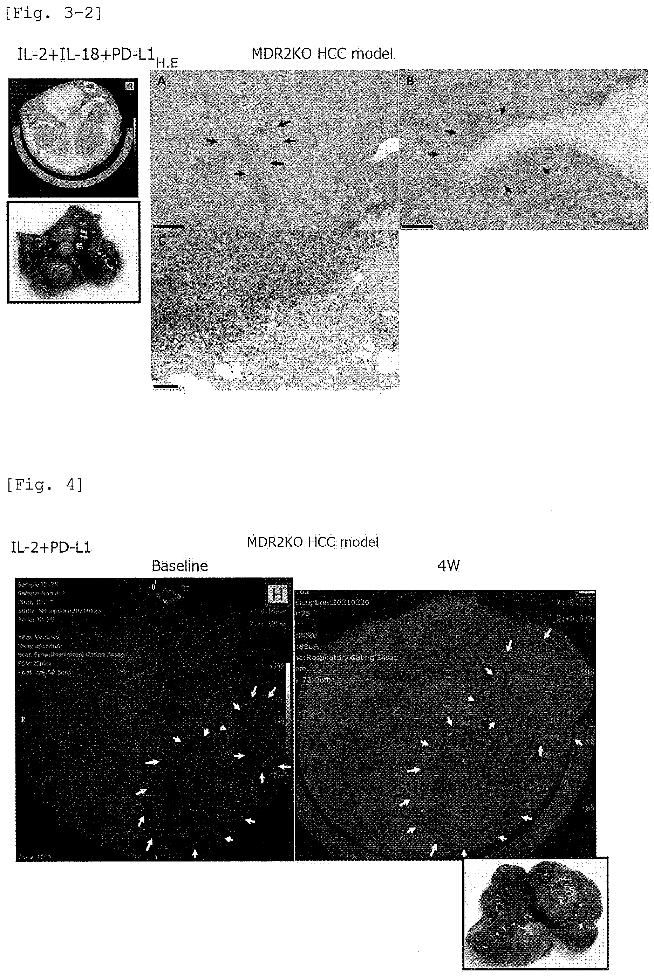

As a result of HE staining of liver tissue sections, hepatocellular cancer accompanied by a glandular duct-like structure was found in the liver parenchyma of the Control group, but infiltration of inflammatory cells around the tumor was not clear (Fig. 2-2, A - C). On the other hand, hepatocellular cancer accompanied by a glandular duct-like structure and necrotic tissues were found in the liver parenchyma in the IL-2+IL-18+PD-L1 administration group. In addition, infiltration of inflammatory cells around the tumor was remarkable, and lymphocytes and mononuclear cells were found. Furthermore, infiltration of inflammatory cells was found in the tumor, and images of hepatocyte regeneration surrounding necrotic tissues and lymphocyte infiltration were found (Fig. 3-2, A - C).

### (2) Decrease in tumor marker expression

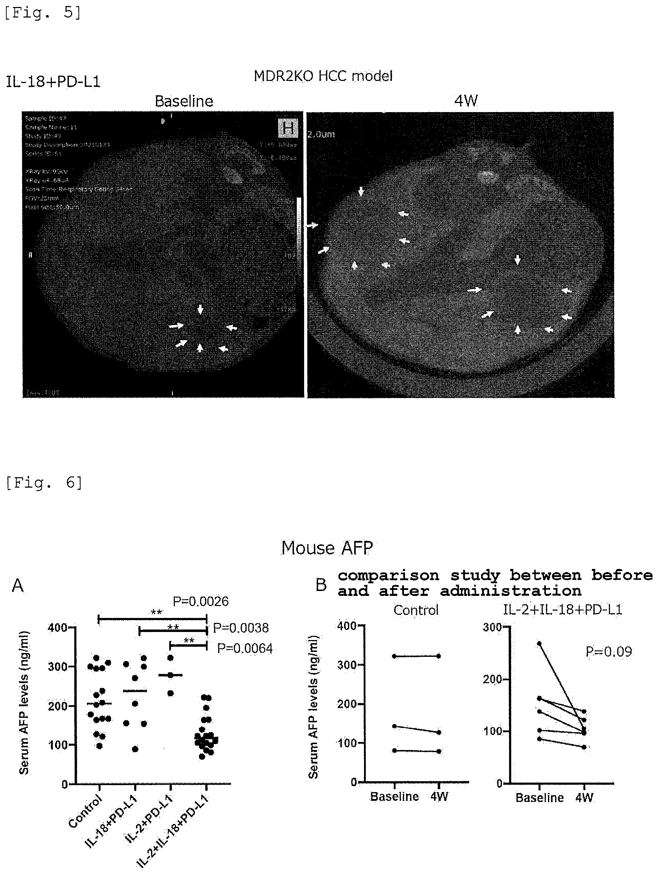

Blood samples were collected from the mice of each group 4 weeks after the start of administration, and the serum AFP level was measured by a conventional method. As a result, a significant decrease in the AFP level was found in the IL-2+IL-18+PD-L1 administration group, as compared with the IL-2+PD-L1 administration group and the IL-18+PD-L1 administration group (Fig. 6A). A comparison between before and after drug administration showed a AFP value-decreasing tendency in the IL-2+IL-18+PD-L1 administration group due to the combined administration (Fig. 6B).

### (3) Cytokine production enhancing effect

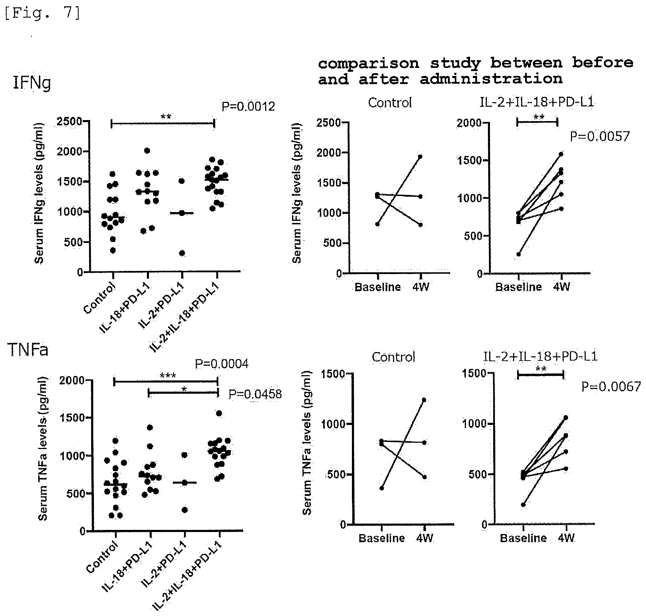

The IFN-γ and TNF-α levels in the serum of each drug administration group were measured and compared 4 weeks after the start of administration. As a result, two-agent combination (IL-2+PD-L1 administration group and IL-18+PD-L1 administration group) could not significantly increase the production of IFN-γ or TNF-α, but three-agent combination (IL-2+IL-18+PD-L1 administration group) significantly increased the production of FN-γ and TNF-α (Fig. 7, left). A comparison between before and after drug administration showed a significant increase in the serum levels of both IFN-γ and TNF-α in the IL-2+IL-18+PD-L1 administration group due to the combined administration (Fig. 7, right).

### (4) Contribution of T cells and NK cells to therapeutic effect of three-agent combination

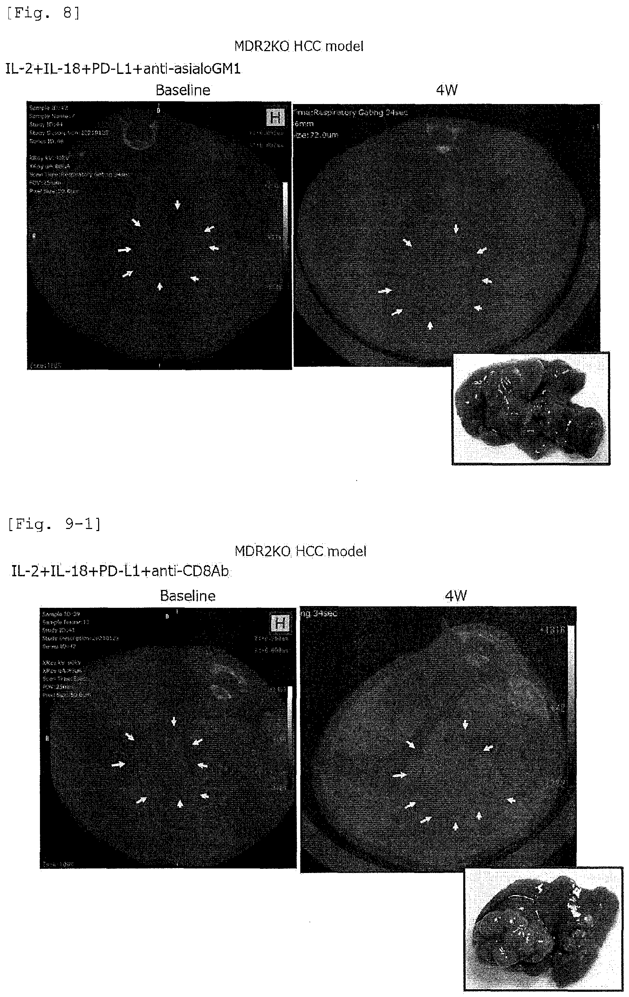

An antibody against asialoGM1, which is a glycolipid expressed on the surface of NK cells, was administered in addition to IL-2+IL-18+PD-L1 to remove NK cells. As a result, it was clarified by image diagnosis of before and after drug administration that the tumor size increases (Fig. 8). This suggests that NK cells play a role in the tumor growth suppressive effect by three-agent combination.

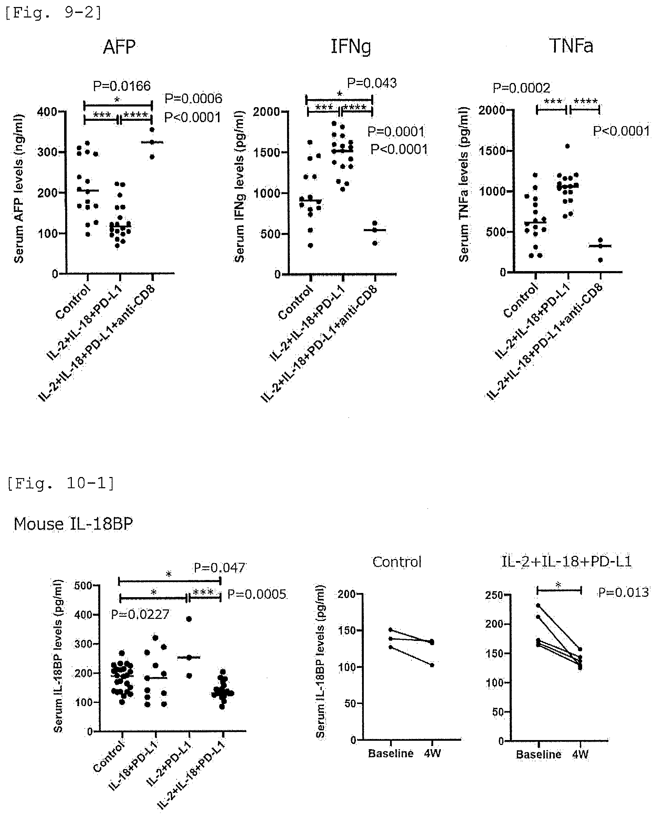

Also, an antibody against CD8 expressed on the surface of killer T cells (CTL) was administered in addition to IL-2+IL-18+PD-L1 to remove CTL. As a result, it was clarified by image diagnosis of before and after drug administration that the tumor size increases (Fig. 9-1). Furthermore, a comparison of the AFP, IFN-γ, TNF-α levels in the serum 4 weeks after the start of administration showed a significant increase in the AFP level and a significant decrease in the IFN-γ, TNF-α levels in the IL-2+IL-18+PD-L1+anti-CD8 antibody administration group as compared with the IL-2+IL-18+PD-L1 administration group (Fig. 9-2). This shows that CTL plays an important role in the tumor growth suppressive effect by three-agent combination, at least via an inflammatory cytokine production enhancing action.

### (5) Relationship to IL-18BP

Serum was collected from the mice in each drug administration group 4 weeks after the start of administration, and the IL-18BP level in the serum was measured and compared. As a result, the IL-18BP level in the serum of Mdr2 KO mouse increased and it was suggested that IL-18BP is highly expressed in the cancer microenvironment. Two-agent combination (IL-2+PD-L1 administration group and IL-18+PD-L1 administration group) could not decrease the IL-18BP level and, although the N number was small, the IL-18BP level rather increased by drug administration in the IL-2+PD-L1 administration group. In contrast, the IL-18BP level could be significantly decreased in the IL-2+IL-18+PD-L1 administration group (Fig. 10-1, left). A comparison between before and after drug administration showed a significant decrease in the IL-18BP level by combined administration in the IL-2+IL-18+PD-L1 administration group (Fig. 10-1, right).

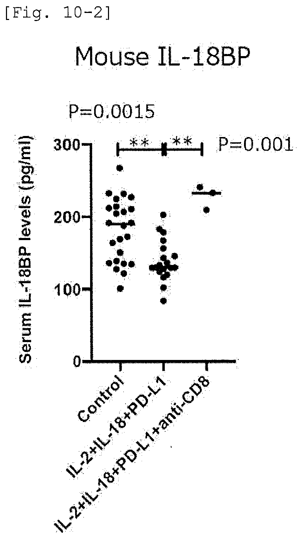

A IL-18BP expression decreasing action by three-agent combination became ineffective by the administration of an anti-CD8 antibody (Fig. 10-2). This suggests that CTL plays an important role in the IL-18BP expression decreasing action.

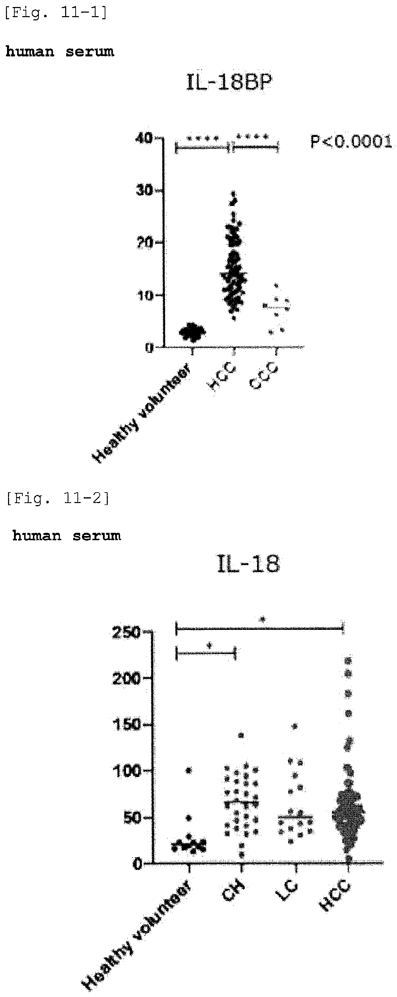

The IL-18BP level of the sera collected with informed consent from hepatitis C virus (HCV)-positive hepatocellular cancer patients (69 patients) and intrahepatic bile duct cancer patients (8 patients), who visited Tokyo Metropolitan Komagome Hospital, and 24 healthy volunteers was measured. As a result, it was clarified that the serum IL-18BP level markedly increased in the hepatocellular cancer patients as compared with the healthy individuals and the intrahepatic bile duct cancer patients (Fig. 11-1). In addition, up to 4-fold or more differences in the IL-18BP level were observed among the hepatocellular cancer patients.

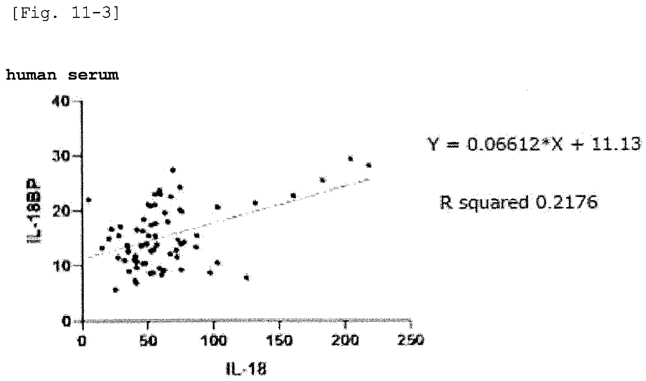

On the other hand, the IL-18BP level of the sera collected from the aforementioned hepatocellular cancer patients (69 patients) and 24 healthy volunteers was measured. As a result, the serum IL-18 level significantly increased in the hepatocellular cancer patients as compared with the healthy individuals (Fig. 11-2; the results of the IL-18 level of the sera collected from chronic hepatitis patients (30 patients) and cirrhosis patients (15 patients) are also shown). However, no correlation was found between the serum IL-18 level and the serum IL-18BP level in the hepatocellular cancer patients (Fig. 11-3).

### [Industrial Applicability]

The combination agent of the present invention is extremely useful in that it can be a combination cancer immunotherapeutic agent effective for intractable liver cancer for which monotherapy with an immune checkpoint inhibitor or combined use thereof with IL-18 is ineffective. In addition, it becomes possible to predict cancer patients for whom the present combination agent will be more markedly effective, by performing companion diagnostic to measure serum IL-18BP levels.

## Claims

1. A combination of an immune checkpoint inhibitor which is an anti-PD-L1 antibody or an anti-PD-1 antibody, interleukin-18 (IL-18), and a T cell growth factor which is IL-2, for use in a method of treating a liver cancer resistant to monotherapy with an immune checkpoint inhibitor or a combination therapy with an immune checkpoint inhibitor and IL-18.

2. The combination of the immune checkpoint inhibitor, IL-18, and the T cell growth factor for use according to claim 1, which is administered to a subject with a serum IL-18 binding protein (IL-18BP) level of not less than 10 pg/mL.

## Patentansprüche

1. Kombination aus einem Immun-Checkpoint-Inhibitor, bei dem es sich um einen anti-PD-L1-Antikörper oder einen anti-PD-1-Antikörper handelt, Interleukin-18 (IL-18) und einem T-Zell-Wachstumsfaktor, bei dem es sich um IL-2 handelt, zur Verwendung in einem Verfahren zur Behandlung eines Leberkrebses, der gegenüber einer Monotherapie mit einem Immun-Checkpoint-Inhibitor oder einer Kombinationstherapie mit einem Immun-Checkpoint-Inhibitor und IL-18 resistent ist.

2. Kombination aus dem Immun-Checkpoint-Inhibitor, IL-18 und dem T-Zell-Wachstumsfaktor zur Verwendung gemäß Anspruch 1, die einem Patienten mit einem Serumspiegel des IL-18-Bindungsproteins (IL18BP) von nicht weniger als 10 pg/ml verabreicht wird.

## Revendications

1. Combinaison d'un inhibiteur de point de contrôle immunitaire qui est un anticorps anti-PD-L1 ou un anticorps anti-PD-1, d'interleukine-18 (IL-18) et d'un facteur de croissance des lymphocytes T qui est IL-2, destinée à être utilisée dans un procédé de traitement d'un cancer du foie résistant à une monothérapie avec un inhibiteur de point de contrôle immunitaire ou à une thérapie combinée avec un inhibiteur de point de contrôle immunitaire et IL-18.

2. Combinaison de l'inhibiteur de point de contrôle immunitaire, d'IL-18 et du facteur de croissance des lymphocytes T destinée à être utilisée selon la revendication 1, qui est administrée à un sujet avec un taux sérique de protéine de liaison à l'IL-18 (IL-18BP) d'au moins 10 pg/mL.
